# EUROPEAN PATENT SPECIFICATION

(11) **EP 2 995 627 B1**
(45) Date of publication and mention of the grant of the patent: **28.12.2016**
(21) Application number: 14184168.4
(22) Date of filing: 09.09.2014
(51) Int. Cl.: C08B 37/08, A61K 47/48

(54) **Synthesis of nanoparticular gene carrier systems based on chitosan**
Synthese nanopartikulärer Genträgersysteme auf Basis von Chitosan
Synthèse de systèmes nanoparticulaires transporteurs de gènes à base de chitosane

(43) Date of publication of application: 16.03.2016
(73) Proprietor: Istanbul Universitesi Rektörlügü, 34452 Istanbul (TR); Gök, Mehmet Koray, 34320 Istanbul (TR); Pabuccuoglu, Saadet, 34320 Istanbul (TR); Cevher, Erdal, 34116 Istanbul (TR); Demir, Kamber, 34320 Istanbul (TR); Pabuccuoglu, Serhat, 34320 Istanbul (TR)
(72) Inventor: Gök, Mehmet Koray, 34320 Istanbul (TR); Pabuccuoglu, M Saadet, 34320 Istanbul (TR); Cevher, Erdal, 34116 Istanbul (TR); Demir, Kamber, 34320 Istanbul (TR); Pabuccuoglu, Serhat, 34320 Istanbul (TR)
(74) Representative: Mutlu, Aydin

(56) References cited:
- FR-A1- 2 754 823
- KIM J H ET AL: "Synthesis and properties of diethylaminoethyl chitosan", POLYMER, ELSEVIER SCIENCE PUBLISHERS B.V, GB, vol. 34, no. 9, 1 May 1993 (1993-05-01), pages 1952-1957, XP024120714, ISSN: 0032-3861, DOI: 10.1016/0032-3861(93)90441-C [retrieved on 1993-05-01]

## Description

### Technical Field of the Invention

The present invention relates to novel biocompatible compounds of chitosan as well as amine modified(MChi), and reducible-amine modified forms (ARMChi) thereof for use as gene carrier systems. The present invention also pertains to nanoparticle forms of the chitosan compounds according to the present invention which provide higher efficiency and lower toxicity for transfection of genes in a biotechnological therapy.

### Background of the Invention

The main purpose of the gene therapy is to overcome the genetic abnormality via transferring the genetic material to the targeted site of the patient. The pre-clinical or clinical gene therapy researches have been improved rapidly in order to provide the regeneration of the defective biological functions or maintenance of homeostasis for the last fifteen years.

Nowadays, gene therapy provides great hope especially for the treatment of genetic diseases, cancer, cardiovascular diseases and viral infection. Gene therapy aims not only to treat the diseases but also aims to transport recombinant genetic materials to the nucleus allowing the gene expression wherein activated or deactivated protein synthesis occurs. Transfection (gene transfer) is the transfer of a gene to the nucleus of another cell via various methods, such as electroporation, gene microinjection, viral gene transfer, non-viral gene transfer and introduction thereof into the cell's DNA.

The carrier systems, which deliver gene for the gene expression, are called "vector". The vectors are divided into two main groups as viral and non-viral. The viral vectors carry out effective transfection due to their structures but the use of these vectors is limited because of their weak characteristics, such as cytotoxicity, viral combination etc. Because of their said negative characteristics, the researchers have tended to use non-viral vectors.

The researchers have developed new, targeted and non-viral gene carrier systems to be widely used in many applications such as transgenesis, process of introducing an exogenous gene into a living organism and gene therapy. However, some problems have been encountered such as the difficulties in the targeting of the gene carrier system, enzymatic degradation of the particles, and cytotoxicity of chemical agents etc. Currently, the studies focus on solving these problems.

Nowadays, the non-viral vectors are used in a rate of less than 25 percent in clinical treatments, and rest of the use is directed to the viral vectors. Since 1989 when the first gene therapy was performed, this approach is applied to more than 3000 patients by means of 600 procedures. (AMIJI, M.M., 2005, Polymeric Gene Delivery, CRC Press, London, 0-8493-1934-X.; KWON, G.S., 2005, Polymeric Drug Delivery, Taylor & Francis Group, Boca Raton, 978-0-8247-2532-7). The non-viral vector systems are not effective enough for the gene transfer, therefore there is no commercial products of these systems. These systems are divided into two groups as lipophilic and polymeric vectors. The cationic liposomes which are used before and during clinical studies, have the same toxicity values as viral vectors. (TAIRA, K., KATAOKA, K., NIIDOME, T., 2005, Non-Viral Gene Therapy, Springer, 4-431-25122-7; WONG, S.Y., PELET, J.M., PUTNAM, D., 2007, Polymer Systems For Gene Delivery-Past, Present And Future, Progress İn Polymer Science, 32, 799-837).

Various polymeric vectors, which are proved to be as efficient as viral vectors and some cationic carrier systems which are capable of forming ion complexes with a gene in anionic structure, have been developped in prior art (LUO, D.; SALTZMAN, W.M., 2000, Synthetic DNA delivery systems, Nat. Biotechnol. 18 (1), 33-37, VORHIES, J.S., NEMUNAITIS, J.J., 2009, Synthetic vs. Natural/Biodegradable Polymers For Delivery of Shrna-Based Cancer Therapies. Methods in Molecular Biology, Macromolecular Drug Delivery, 480, 11-29). The common target of these studies is the synthesis of the polymeric carrier systems, which are as effective as viral vectors and have low toxicity. The most important advantage of polymeric vectors and polypeptides is the enhancement of their efficacy by allowing various modifications which improve the genetic material release characteristics of the polymers to the targeted site (THOMAS, M., KLIBANOV, A.M., 2003, Non-Viral Gene Therapy: Polycation-Mediated DNA Delivery, Applied Microbiology And Biotechnology, 62, 27-34). The other advantages of the cationic polymers are low toxicity, immune response sensitivity, ease in production and stability (Mandke, R.; Singh, J. 2012, Cationic nanomicelles for delivery of plasmids encoding interleukin-4 and interleukin-10 for prevention of autoimmune diabetes in mice. Pharm. Res. 29, 883-897; Wu, G.C.; Zhou, F.; Ge, L.F.; Liu, X.M.; Kong, F.S. 2012, Novel Mannan-PEG-PE modified bioadhesive PLGA nanoparticles for targeted gene delivery. J. Nanomater. 2012, 1-9).

The transgenic polymers are preferred generally in gene delivery due to their ability to be easily modified. The transgenic polymers are divided into two groups; synthetic transgenic polymers and the natural transgenic polymers. The natural polymers generally used in gene delivery are the poly(aminoacids), such as poly-L-lysine (PLL), polyornithine, polyarginine etc., chitosan, dextran, collagen, gelatin and their modified derivatives (TIERA, M.J.; WINNIK, F.M.; 2006, FERNANDES, J.C., 2006, Synthetic and natural polycations for gene therapy: State of the art and new perspectives. Curr. Gene Ther. 6, 59-71; HOSSEINKHANI, H.; AZZAM, T.; TABATA, Y.; DOMB, A., 2004, Dextran-spermine polycation: an efficient nonviral vector for in vitro and in vivo gene transfection. Gene Ther. 11, 194-203; CHEVALLAY, B.; HERBAGE, D., 2000, Collagen-based biomaterials as 3D scaffold for cell cultures: Applications for tissue engineering and gene therapy. Med. Biol. Eng. Computing 38, 211-218; KAUL, G.; AMIJI, M. 2005, Tumor-targeted gene delivery using poly (ethylene glycol)-modified gelatin nanoparticles: in vitro and in vivo studies. Pharm. Res., 22, 951-961; KOMMAREDDY, S.; AMIJI, M., 2007, Antiangiogenic gene therapy with systemically administered sFlt-1 plasmid DNA in engineered gelatin-based nanovectors. Cancer Gene Ther. 14, 488-498). Similarly, according to Amiji, (AMIJI, M.M., 2005, Polymeric Gene Delivery, CRC Press, London, 0-8493-1934-X.) the synthetic transgenic polymers can be classified as non-biodegradable synthetic transgenic polymers (polyethylene imine (PEI), polyethylene glycol (PEG) conjugates etc.), biodegradable transgenic synthetic polymers (poly(β-aminoesters (PBAE), polyamido amines, poly-imidazole etc.), polyethylene oxide/polypropylene oxide copolymers and polymeric polyethylene oxide, and polyalkyl cyanoacrylate nano-microspheres.

Chitosan is one of the prominent non-viral gene carriers in gene therapy. Chitosan, which is a cheap, biocompatible, biodegradable and non-toxic cationic polymer, is a linear polysaccharide, composed of glucosamine and N-acetyl glucosamine units linked by β (1-4) glycosidic bonds and a partially deacetylated product of the natural polysaccharide chitin (BSME, W.C.S. Scaleless Dieting: The Essential Survival Kit for the Overweight, Obese and Diabetics; AuthorHouse: Bloomington, 2011. ELKINS, R.; HENNEN, W.J. Chitosan; Woodland Publishing, 1996). Besides these properties, the molecule itself and its degradation products display other excellent biological properties such as immunological, antibacterial and wound-healing activity. Various possible modification reactions such as nitration, phosphorylation, sulphation, thiolation, acylation, hydroxyalkylation, graft polymerization, amination, combination of chitosan derivatives with cyclodextrins etc. can easily be applied. Chitosan has high positive charge density due to d-glucosamine units in its structure. Thus, amine groups on the chitosan are protonated in acidic and neutral pH and chitosan forms polyelectrolyte complexes with the negatively charged DNA (AMIJI, M.M., 2005, POLYMERIC GENE DELIVERY, CRC PRESS, LONDON, 0-8493-1934-X, CHO, Y.-W.; HAN, S.-S.; KO, S.-W. 2000, PVA CONTAINING CHITO-OLIGOSACCHARIDE SIDE CHAIN. POLYMER, 41, 2033-2039, SARANYA, N.; MOORTHI, A.; SARAVANAN, S.; DEVI, M.P.; SELVAMURUGAN, N., 2011, Chitosan and its derivatives for gene delivery. Int. J. Biol. Macromol. 48, 234-238). Nowadays, chitosan carriers are more reliable non-viral carrier polymers than cationic lipids and other non-viral cationic polymers (i.e. PEI or polyamidoamine dendrimers) due to its low toxicity and immunogenicity. Therefore, recently, chitosan and its derivatives are recognized as safe and efficient cationic carriers for gene delivery (AMIJI, M.M., 2005, POLYMERIC GENE DELIVERY, CRC PRESS, LONDON, 0-8493-1934-X, PARK, S.; JEONG, E.J.; LEE, J.; RHIM, T.; LEE, S.K.; LEE, K.Y., 2013, Preparation and characterization of nonaarginine-modified chitosan nanoparticles for siRNA delivery. Carbohyd. Polym. 92, 57-62, JEEVITHA, D.; AMARNATH, K., 2013, Chitosan/PLA nanoparticles as a novel carrier for the delivery of anthraquinone: Synthesis, characterization and in vitro cytotoxicity evaluation. Colloid Surf. B, 101, 126-134, OPANASOPIT, P.; SAJOMSANG, W.; RUKTANONCHAI, U.; MAYEN, V.; ROJANARATA, T.; NGAWHIRUNPAT, T., 2008, Methylated N-(4-pyridinylmethyl) chitosan as a novel effective safe gene carrier. Int.J. Pharm. 364, 127-134, SAJOMSANG, W.; GONIL, P.; RUKTANONCHAI, U.R.; PETCHSANGSAI, M.; OPANASOPIT, P.; PUTTIPIPATKHACHORN, S., 2013, Effects of molecular weight and pyridinium moiety on water-soluble chitosan derivatives for mediated gene delivery. Carbohyd. Polym. 91, 508-517, ALVES, N.; MANO, J., 2008, Chitosan derivatives obtained by chemical modifications for biomedical and environmental applications. Int. J. Biol. Macromol. 43, 401-414, ZOHURIAAN-MEHR, M.J., 2005, Advances in chitin and chitosan modification through graft copolymerization: A comprehensive review. Iran Polym. J. 14, 235-265). However, because of the lower efficiency in binding and transfecting the genetic material, there is a considerable need in the art to eliminate the limitations concerning chitosan by way of new forms and suitable modification processes in line with the objectives scrutinized in the detailed description. These objectives have been achieved through the novel products and methods for producing the same as disclosed in the appended claims.

### Brief Description of the Figures

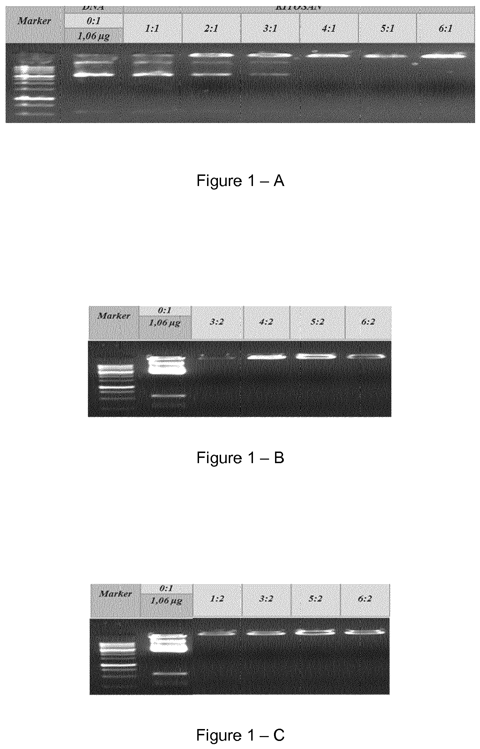

**Figure 1 - A**: Gel electrophoresis image of complexes formed with Chi nanoparticles.
**Figure 1** **- B:** Gel electrophoresis image of complexes formed with nMChi nanoparticles according to the present invention.
**Figure 1** **- C:** Gel electrophoresis image of complexes formed with nARMChi nanoparticles according to the present invention.

### Detailed Description of the Invention

The current invention is related to the synthesis and characterization of biocompatible modified chitosan (MChi) and reducible-modified chitosan structures (RMChi) which can be further modified to an amine modified form (ARMChi), which are found as suitable gene carrier systems. The present invention also relates to the application of these modified compounds in biotechnology by way of realizing the said compounds in the form of nanoparticles for administering a biologically useful genetic material to a host organism.

More particularly, the current invention is related to the modified compounds of chitosan as mentioned above as well as novel use thereof as gene carrier vectors in cell transfection, gene transfer and gene therapies.

Some terms and their abbreviations used in the context of the present description are given below:
Chi : As used herein, the abbreviation "Chi" refers to chitosan.
MChi : As used herein, the abbreviation "MChi" refers to amine modified chitosan which is modified with amine component on the chitosan chain.
RMChi : As used herein, the abbreviation "RMChi" refers to reducible chitosan which is modified by using a disulfide component on Chi.
ARMChi : As used herein, the abbreviation "ARMChi" refers to amine modified reducible chitosan which is modified with amine component on the RMChi.
nMChi: As used herein, the abbreviation "nMChi" refers to nanoparticles which are obtained from MChi.
nARMChi: As used herein, the abbreviation "nARMChi" refers to nanoparticles which are obtained from ARMChi.
gnMChi: As used herein, the abbreviation "gnMChi" refers to nanoparticles which are obtained from MChi via complexing with a pDNA.
gnARMChi: As used herein, the abbreviation "gnARMChi" refers to nanoparticles which are obtained from ARMChi via complexing with a pDNA.

The inventors, in studies they have conducted, aimed to synthesize modified chitosan nanoparticles not synthesized before, forming complexes with the conventional plasmid DNAs (pDNA; i.e. green fluorescense protein gene; GFP) at a desirable rate and appropriate zeta potential value along with desirable particle size values without displaying high toxicity.

At the end of studies made, the inventors have observed that modified chitosan having desired features can basically be obtained by using N-(2-hydroxyethyl)ethylenediamine (HE-EDA) via the modification of the amine group on Chitosan.

Therefore, according to the first aspect of the current invention, there is provided an amine modified chitosan molecule that is modified by using N-(2-hydroxyethyl) ethylenediamine (HE-EDA). The inventors have duly noted that treatment of the conventionally available natural chitosan polymer with HE-EDA prominently increases the amine groups on chitosan's linear polymeric chain. This gives rise to novel polymers efficiently binding and delivering of genes to a target host as demonstrated in the Examples. These novel compounds are simply referred to as MChi. In an exemplary method for producing said compound, chitosan may for instance be dissolved in a solvent and then HE-EDA can be poured onto this solution followed by precipitation and drying of MChi. The solvent used is preferably acetic acid. An exemplary reaction scheme is shown below to show the amine modification of the Chitosan polymer by using HE-EDA:

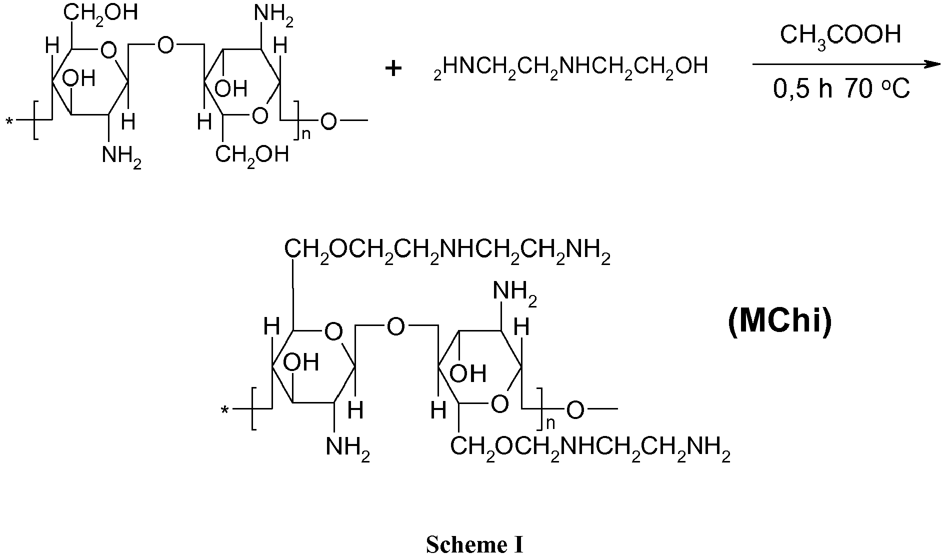

In a second aspect of the present invention, Chitosan or modified form thereof (MChi) can be brought into a reducible form by way of treating the same with a disulphur compound such as allyl disulfide which results in modified reducible chitosan moieties. Both of the modified reducible chitosan moieties either derived from MChi or directly from the Chitosan shall hereinafter be simply referred as RMChi. These reducible compounds are found to be advantageous as intermediates for further modifications to increase free amine content on the linear chain of chitosan as noted in the following explanations. In an exemplary method for obtaining these intermediates, Chi or modified form thereof (MChi) is initially treated with a gelation reaction which is then treated with a disulphur compound as mentioned above, followed by precipitation and drying. Using of an initiator such as Cerium-ammonium nitrate (CAN) in the procedure can be advantageous to allow formation of free radical groups. The gelation reaction may be conducted at a temperature for instance between 60 and 85 °C, and more preferably 70-75 °C.

In a third aspect of the present invention, the RMChi moieties can advantageously be used as an intermediate to produce amine modified reducible chitosan (ARMChi) which is also found as an efficient gene carrier system in the context of the present invention. Said RMChi is treated with ethylene diamine (EDA) that increases the free amine content. In an exemplary method, RMChi is dissolved in a solvent solution, preferably comprising acetic acid, treated with ethylene diamine (EDA) and then recovered by way of known methods such as dialysis and lyophilization. The inventors noted that disulfide bonds of RMChi that is used as an intermediate in the context of the present invention can be protected, for instance by using hydrogen peroxide which gives rise to higher amounts of free amine groups and therefore better binding and transfection properties. The reaction schemes to obtain amine modified reducible chitosan moieties (ARMChi) starting either from Chi or MChi is given below:

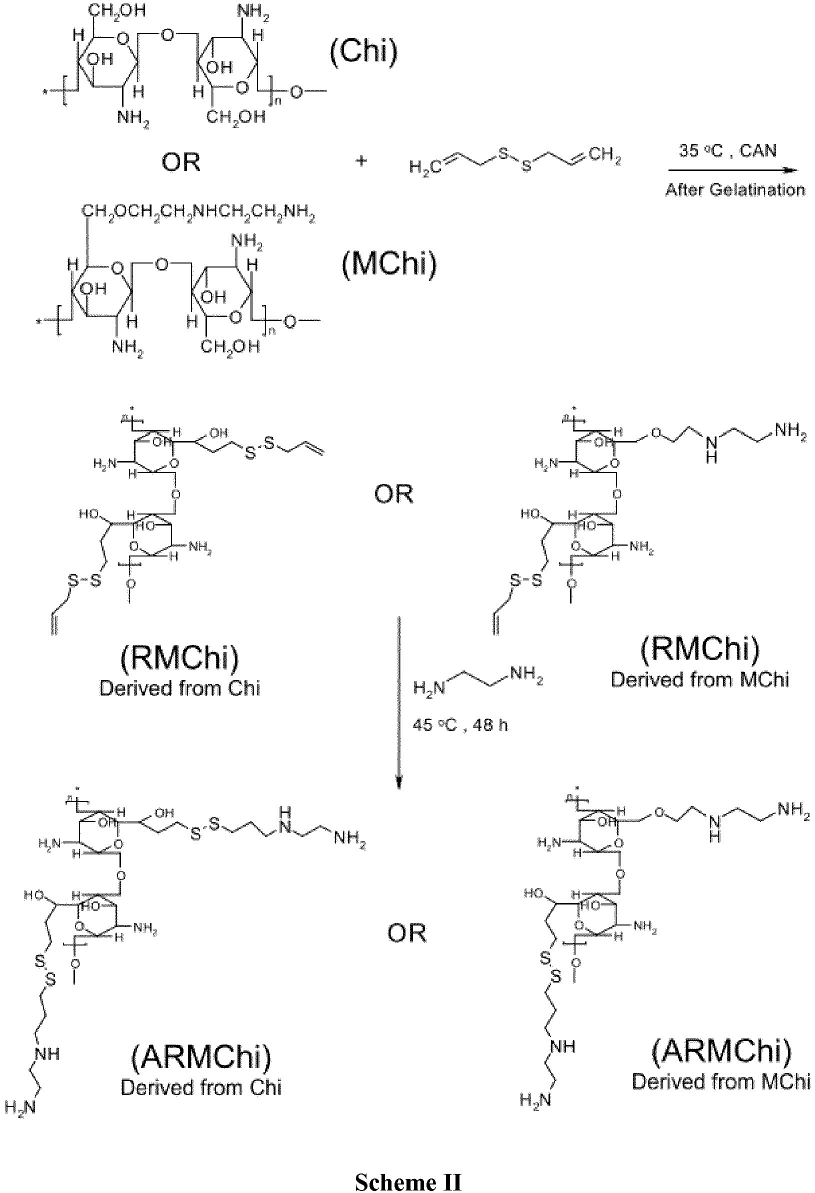

In a fourth aspect of the present invention, provided are nanoparticles comprising MChi or ARMChi moieties as shown above, or a mixture thereof serving as carrier systems. The inventors have found out that said MChi and ARMChi structures can advantageously be converted into a nanoparticular form. The inventors also surprisingly noted that the nanoparticular forms according to the present invention require less amount of chitosan derivatives for carrying a particular amount of genetic material, hence ensuring a higher efficiency of transfection. That is the same amount of pDNA can form complexes with less amount of polymeric nanoparticular system as compared to controls including chitosan. Such nanoparticles are prepared from amine modified chitosan (MChi) or amine modified reducible derivative of chitosan (ARMCHi) according to the present invention by way of a method known in the art such as ionic gelation method.

According to the experimental studies as discussed in the examples, the nanoparticles that are obtained with suitable particle sizes advantageously provide an efficiency of up to several folds with their higher affinity and binding properties to DNA. In an exemplary method for obtaining said nanoparticles, MChi and/or ARMChi may for instance be dissolved in a suitable solvent and then treated with gel forming compound, such as a crosslinking compound, more preferably with sodium tripolyphosphate (TPP) followed by drying. The solution used in this method may further be incorporated a disulfide protecting compound such as hydrogen peroxide for ARMChi.

For the MChi containing nanoparticles (nMChi), preferred particle size is between 100 - 210 nm, and preferably about 100 nm which gives excellent results. The polydispersity value of amine modified chitosan nanoparticles is 0.100-0.800, preferably 0.300-0.750, and more preferably the polydispersity value is about 0.400. Moreover the zeta potential value of amine modified chitosan nanoparticles can be between 1.50-50 mV, preferably 8-40 mV, and more preferably 39-49.5 mV.

For the ARMChi containing nanoparticles (nARMChi), the particle size is between 100-210 nm, more preferably about 110 nm. In this case, the polydispersity value of reduced modified chitosan nanoparticles is 0.100-0.800, preferably 0.200-0.500, more preferably 0.220-0.480, and most preferably the polydispersity value is about 0.220.

The zeta potential value of amine modified reduced chitosan nanoparticles is between 1.5 to 50 mV, but preferably is 34-55 mV, and more preferably 34-50 mV.

An advantageous feature of the complexes according to the present invention is that, for the modified chitosan nanoparticles (gnMChi), a polymer:pDNA ratio of 1:1 would be sufficient unlike the unmodified chitosan which require much more chitosan polymer for delivering same amount of DNA. In the case of nanoparticles obtained by further modification of reduced modified chitosan (gnARMChi) a polymer:pDNA ratio of 1:2 would be sufficent for form forming complexes with all pDNA.

The present invention is described further by way of non-limiting example hereinbelow.

### EXAMPLE

### Material and Methods

The monomers; low molecular weight Chitosan (Chi), which was used for the MChi and ARMChi sythesis, was purchased from Sigma-Aldrich (USA).

N-(2-hydroxyethyl)ethylenediamine (HE-EDA), used for the MChi synthesis, was purchased from Sigma-Aldrich (USA). Ethylenediamine (EDA), used for the ARMChi sythesis, was purchased from Sigma-Aldrich (USA).

Glacial acetic acid, as the solvent, and acetone, as the precipitant, were obtained from Merck (Germany). Cerium-ammonium nitrate (CAN), as initiator, and allyl disulphide, as thiol component, which were used for the ARMChi sythesis, were purchased from Sigma-Aldrich (USA). For the nanoparticle carrier system synthesis, glacial acetic acid and deionized water are used as solvent and sodium tripolyphosphate (TPP), as crosslinker, were purchased from Sigma-Aldrich (USA).

For the preparation of lysogen medium; yeast extract (Nitragen Based, (-) free amino acids), Triptone (Bacto) and sodium chloride (for the molecular biology) (Sigma), for the preparation of the competent bacteria cells; calcium chloride (CaCl₂) (for the cell culture test) (Sigma) and glycerol (for the molecular biology) (Sigma) were used.

Tris base (Sigma; USA), glacial acetic acid (Merck; Germany) and EDTA (Sigma; USA) for the preparation of Tris-Acetate-EDTA buffer (TAE, pH 8.0), agarose, ethidium bromide (for the molecular biology) and bromphenol blue (Sigma; USA) were used for the gel electrophoresis experiments.

For the cell culture and gene transfection tests, Dulbecco modified eagle medium (DMEM) were used and for the preparation of this medium, L-glutamate, sodium pyruvate, sodium bicarbonate (NaHCO₃), peniciline and streptomycin, which were obtained from Sigma; USA, were used. Besides that, fetal calf serum (FCS) (Sigma; USA) was used for the FCS-containig DMEM (FCS+DMEM). For the cell counts, trypan blue was used as the colorant (Sigma; USA).

### The Methods of Modified Chitosan (MChi) and Reducible-Modified Chitosan (RMChi) Synthesis and Reaction System

The synthesis of MChi was carried out via modification of the amine component of methylol groups in HE-EDA in order to increase the amine amount on the chitosan molecule. For this purpose, the chitosan molecule was dissolved in 4% acetic acid solution. N-(2-hydroxyethyl)ethylenediamine (HE-EDA) was poured into the chitosan solution (0,5 moles HE-EDA per 1 mole glycosamine -) and mixed at 70 °C for 30 min. In the end of the period for the purification, the MChi was precipitated in cold aceton solution, dried under vacuum at 40 °C and stored at +4 °C.

The synthesis of RMChi compound is realized via the modification of the Chi compound, the synthesis thereof is described in the paragraph above, with a disulphur compound (allyl disulfide) under nitrogen atmosphere. First, for the gelatinization of Chi, a mixture of 4 g Chi and 108 mL water was stirred at 70-75 °C for one hour. Then the mixture was cooled to at 35 ±1 °C and treated with CAN (0.005 mol/L) as initiator for 15 min to allow the formation of free radical groups. After that, diallyl-disulfide component (1 mole diallyl-disulfide / per 1 mole glucosamine) was added into the reactor and mixed for 3 hours. The purification, at the end of the reaction time, was realized with the precipitation of the mixture in the presence of excessive cold-methanol. Following this step it is filtrated and washed with ethyl alcohol. The obtained product is dried at 50 °C in the vacuumed incubator.

RMChi product was modified with the ethylene diamine (EDA) for increasing the free amine content on the chitosan. RMChi product was dissolved in acetic acid solution (1% v/v) and then the EDA solution (1,1 mole EDA / per 1 mole glucosamine) was dropped into this solution. In this reaction step, the hydrogen peroxide solution (0,3% v/v) was added for the inhibition of the degradation of the disulfide bonds. The reaction was occurred at 45 °C for 48 h. At the end of the reaction, the product (ARMChi) was dialysed with dialysis socks (Medicell International Ltd, Size 3 Inf Die 20/32-15,9mm, MWCO-12-14000 Daltons) at 10±1 °C for the purification and then lyophilized at -30±1 °C on 0,01 mbar (Lyovac GT2E, Steris, Germany). ARMChi was stored at +4 °C.

### The Characterization of Modified Chitosan Based Products

### Fourier Transform Infrared Spectroscopy (FTIR) Analyses

The structure of synthesized MChi and ARMChi synthesized were identified by Fourier Transform Infrared Spectrophotometer (FTIR) technique. The analysis was performed using Digilab Excalibur-FTS 3000 MX model FT-IR apparatus. For FTIR analysis, tablets prepared by dilution where a sample/KBr ratio is of 1/200, is used and recorded at a wavenumber range of 400-4000 cm⁻¹.

### The Preparation of Nanoparticular Carrier Systems From MChi and ARMChi Based Products

The nanoparticular carrier systems from MChi and ARMChi are prepared via the ionic gelation method. The MChi and ARMChi were dissolved into %1 acetic acid solution and diluted with distilled water to be prepared in different concentrations. Similarly, TPP-water solution (%0,1 m/v) was prepared and dropped onto the MChi or ARMChi solutions at a constant stirring rate (250 rpm) for an hour. A hydrogen peroxide solution (0,3% v/v) was added to inhibit the degradation of the ARMChi's disulfide bonds. The nanoparticles (nMChi and nARMChi) are stored at +4 °C.

### The Preparation of Nanoparticles Carrying pDNA

nMChi and nARMChi were treated with the pDNA in different amounts (m/m) (gnMChi, gnARMChi) and analysed.

### Applied Tests and Instruments for nMChi, nARMChi, gnMChi and gnARMChi

### The Analysis of Zeta Potential, Particular Size and Polidispersity Index (PDI)

The zeta potential, particular size and PDI analyses of nMChi, nARMChi, gnMChi and gnARMChi were measured in deionized water at 25 °C with Zetasizer Nano Series (Malvern Instruments, England) instrument. Therefore, the particles sizes of nanoparticles have been measured based on Dynamic Light Scattering (DLS) expressed in terms of Z-average. Polydispersity index of the nanoparticles is an indication of how narrow a Gaussian size distribution would be that could represent the fitted DLS data.

### Gel Electrophoresis Analayses of gnMChi and gnARMChi

Produced complexes are analysed on gel electrophoresis in order to understand the complex formation ability between nanoparticles and pDNA (Cleaver Scientific Ltd., England). The agarose is dissolved by being boiled in Tris-acetate-EDTA (TAE, pH 8.0) solution until clear solution occured. After it is cooled to the room temperature, ethidium bromide (0.5 µg/mL) is added. Then the agarose gel was placed into the electrophoresis tank which has pH 8.0 TAE buffer. Sample:electrophoresis loading buffer in a ratio of 9:1 v/v is loaded on the agarose gel by mixing and without mixing with 6x agarose gel loading dye (bromphenol blue). The electrophoresis was completed in 1 hour under 100 V. After the electrophoresis, the agarose gels are evaluated by photos taken on UV transilluminator.

### Cytotoxicity Studies of nMChi, nARMChi and In Vitro Transfection Studies of gnMChi αnd gnARMChi

### Solutions

DMEM-Stock: For the solution preparation, the powder DMEM containing L-glutamate and sodium pyruvate is used. The stock solution was prepared with 13,38 g DMEM powder, 3,7 g NaHCO₃, 60 mg penicillin and 100 mg streptomycin.

FCS with DMEM (DMEM+FCS): DMEM+FCS medium (200 mL) was prepared with 174 mL DMEM Stock, 20 mL FCS, 2 mL Na-pyruvate, 2 mL L-glutamine and 2 mL non-essential aminoacid. DMEM+FCS medium was consumed within 1 week after preparation because of the low stability of aminoaicds and L-glutamine at +4°C.

FCS without DMEM (DMEM-FCS): DMEM-FCS medium (200 mL) was prepared with 194 mL DMEM Stock, 2 mL Na-pyruvate, 2 mL L-glutamine and 2 mL non-essential aminoacid. DMEM-FCS medium was consumed within 1 week after preparation because of the low stability of aminoacids and L-glutamine at +4°C.

### The Preparation of Primer Ovine Fibroblasts (POF), Primer Ovine Embryonic Fibroblasts (POEF), Human Embryonic Kidney (HEK293) Cells

### Thawing of the Cells and Their Usage;

To be used in the studies, POF and POEF situated between 2 and 10 passages were obtained from sheep ear skin in the Istanbul University Faculty of Veterinary Medicine Department of Reproduction and Artificial Insemination and HEK293 cells provided from Marmara University Faculty of Pharmacy Department of Basic Pharmaceutical Sciences were replicated and used.

Cryovial containing cells were removed from liquid nitrogen and were dissolved in 37 °C water bath. To purify the cells in the cryovial from DMSO, the content were centrifuged with 5 ml of DMEM for 5 min. at 2000 rpm. Then the supernatant were discarded and the cells were dispersed within 1 ml of DMEM. The concentration of POF, POEF and HEK293 cells obtained is measured by hemacytometric method with Thoma cell counting chamber. During counting by hemacytometric method, the viability of cells is also detected by using %0.4 trypan blue. After this procedure, the cells are plated into cell culture dishes at appropriate rate per unit cm² and are allowed to proliferate for 1 day.

The cells replicated and calculated with techniques described above to be used in the studies, are dispensed to petri dishes at appropriate concentration.

### Cytotoxicity Studies via Real Time Cell Analyser (RTCA)

The cytotoxicity studies of nMChi and nARMChi are done with xCELLigence RTCA (Roche, Germany).

The working principle of the apparatus is based on the measurement of electrical impedances of cells which are cultivated in gold plate (E-Plate 96) consisting 96 well, via gold electrodes. RTCA registers the electrical impedance changes which are caused by the cells numerical changes whereby the cell proliferation and viability in real time are registered.

100 µl cell culture medium is added in each 96 weell of E-Plate 96. E-Plate 96 is kept at room temperature for 30 minutes. At the end of the period, E-Plate 96 is placed into the xCELLigence and background value is measured.

Two different cell types: HEK293 and POF cells, are used in cytotoxicity studies. After measuring the background value, the cells (30000) are plated into each well for 30 minutes at room temperature. At the end of the period, E-Plate is placed into the device and waited one day for cell proliferation. After one day, the cells are controlled, then DMEM+FCS medium is removed and DMEM-FCS (200 µL) and nMChi and nARMChi products in varying concentrations are added into each well.

The products were allowed to contact with cells for 4 hours. At the end of 4 hours, DMEM-FCS and the products are removed, DMEM+FCS is added onto the cells and cell proliferations were followed for 72 hours.

### The Transfection Efficiency of Nanoparticular Systems

According to the all results of the analysis mentioned above, the transfection efficiencies of gnMChi and gnARMChi products are assayed in HEK293, POF and POEF cells.

20000 cells thawed are plated into each of 24 well and incubated one day for proliferation at 37°C in CO₂ incubator. At the end of 1 day, cell proliferation is controlled with microscope and DMEM+FCS is removed. The nanoparticular systems and DMEM-FCS are added to the cells and the cells are incubated for 4 hours at 37°C in CO₂ incubator. After 4 hours, consumed DMEM-FCS and the nanoparticule systems are removed and then DMEM+FCS is added into the cells instead. The cells were incubated for 3 days at 37°C in a CO₂ incubator. At the end of 3 days, the transfection efficiencies of nanoparticle carriers meaning cell transfection of GFP coding pDNA were analysed with Olympus IX71 reverse microscope under 460-480 nm fluorescence light. Based on the green fluorescence displayed by cells, transfected cells were determined and the numerical values were obtained by the examination of photographs of the specific regions on wells with the imaging system.

The inventors present the results of the studies related to the synthesis of MChi having desired characteristics and development of nanoparticles using these MChis as well as their remarks hereinbelow.

### Result And Discussion

### FTIR Characterization

The fact that the peaks observed in the chitosan spectrum at 1651 cm⁻¹ and 1599 cm⁻¹ indicating the methylol and amine groups on the chitosan, are observed in the MChi polymer spectrum at 1607 cm⁻¹ as a single broad peak, shows that the modification is on the methylol groups. Moreover, in the RMChi spectrum, the small band at approximately 1648-1638 cm⁻¹ region max at 1639 cm⁻¹) is related to the stretching vibrations resulted from the C=C bond in vinyl groups of allyldisulfide compounds showing that the modification of chitosan is occurred with allyldisulfide compound as expected. In the ARMChi polymer spectrum, the small band at 1639 cm⁻¹ mentioned above related to C=C bond, is shifted to the 1648 cm⁻¹, which shows that the modification of RMChi polymer with EDA is realized as expected.

### The Optimization Results of Nanoparticles

Based on the results on Table 1, although all of the nMChi products are convenient for use in gene transfer, nMChi₅ formulations are determined as the most appropriate nanoparticle formulations having 102,9 nm particle size, 0,404 PDI and 41,9±5,63 mV zeta potential. The fact that almost all of the nanoparticles prepared from modified chitosan based polymers have about 0,400 PDI value is because of the wide range of molecular weight distribution of chitosan in the market.

**Table 1 - The zeta potential, particle size and polydispersity values of nMChi**

| **Nanoparticle** | **Particle Size (nm)** | **Polydispersity** | **Zeta Potential (mV)** |
|---|---|---|---|
| nMChi₁ | 125,2 | 0,497 | 29,8±4,23 |
| nMChi₂ | 104,4 | 0,402 | 43,9±4,43 |
| nMChi₃ | 132,0 | 0,753 | 40,9±3,56 |
| nMChi₄ | 108,9 | 0,405 | 32,2±5,41 |
| nMChi₅ | 102,9 | 0,404 | 41,9±5,63 |
| nMChi₆ | 118,7 | 0,420 | 5,31±3,62 |
| nMChi₇ | 104,8 | 0,366 | 28,8±4,47 |
| nMChi₈ | 114,8 | 0,326 | 43,7±8,89 |
| nMChi₉ | 203,9 | 0,302 | 35,9±3,82 |
| nMChi₁₀ | 126,4 | 0,464 | 30,5±4,41 |
| nMChi₁₁ | 181,3 | 0,431 | 38,1±4,12 |
| nMChi₁₂ | 140,9 | 0,418 | 44,4±5,05 |
| nMChi₁₃ | 136,8 | 0,307 | 26,7±3,98 |
| nMChi₁₄ | 113,3 | 0,390 | 40,6±4,81 |
| nMChi₁₅ | 146,0 | 0,645 | 32,7±4,57 |

As seen in Table 2, although all of the nARMChi products are convenient for using in gene transfer, nARMChi₅ product which has 110,8 nm particle size, 0,221 PDI and 42,7±6,63 mV zeta potential, is more appropriate product than the other nanoparticles. The fact that almost all of the nanoparticles prepared from modified chitosan based polymers have about 0,400 PDI value, is because of the wide range of molecular weight distribution of chitosan in the market.

**Table 2 - The zeta potential, particle size and polydispersity values of nARMChi**

| **Nanoparticle** | **Particle Size (nm)** | **Polydispersity** | **Zeta Potential (mV)** |
|---|---|---|---|
| nARMChi₁ | 176,9 | 0,463 | 44,1±3,45 |
| nARMChi₂ | 177,4 | 0,432 | 45,5±4,57 |
| nARMChi₃ | 202,3 | 0,477 | 51,1±3,84 |
| nARMChi₄ | 144,6 | 0,412 | 42,1±5,31 |
| nARMChi₅ | 110,8 | 0,221 | 42,7±6,63 |
| nARMChi₆ | 148,7 | 0,418 | 45,7±4,38 |
| nARMChi₇ | 160,2 | 0,474 | 45,2±4,01 |
| nARMChi₈ | 156,6 | 0,445 | 40,8±6,79 |
| nARMChi₉ | 114,9 | 0,281 | 40,8±6,79 |

The inventors, based on the studies made on modified chitosans, have discovered that nanoparticles prepared with amine modified chitosans synthesized by using HE-EDA have the desired particle size, polydispersity and zeta potential value.

As noted above, the inventors also conducted studies based on amine modified chitosans, wherein chitosan is treated with a disulphur compound, namely allyl disulphide and reduced said compounds. These reduced compounds (RMChi) are modified by using ethylene diamine (EDA). The product formed (ARMChi) as a result of this modification is purified and brought into a form ready to be used.

The inventors, in order to investigate the complex formation between the nanoparticles of the invention and plasmid DNA, have prepared various formulations. The formed complexes identified based on the nanoparticle types used are: gnChi, gnMChi and gnARMChi. As a result, formulations prepared by the combination of said nanoparticles with pDNA complexes of different ratios are formed. These complexes are analyzed by gel electrophoresis technique and the results thereof are presented below.

### The Gel Electrophoresis Test Results for nMChi₅ and nARMChi₅

Based on the gel electrophoresis results of nMChi and nARMChi, the chitosan is complexed with all of the DNA when pure chitosan:pDNA (a/a) ratio is 4:1 (Figure 1-A). gnMChi₅ formulation however, depending on the increase in amine groups formed as a result of the modification in chitosan structure, at a low rate of 1:1, can form complexes with all pDNA (Figure 1-B). Again, in gnARMChi₅ formulation, it is observed that this rate is decreased even to 1:2 (Figure 1-C).

Consequently, it is required that the polymer:pDNA ratio should be at least 4:1 so that the unmodified chitosan nanoparticles form complexes with all pDNA. On the other hand, the 1:1 ratio is sufficient for nanoparticles prepared with the modified chitosans of the present invention to form complexes with all pDNA.

Accordingly, in an embodiment of the invention, a feature of the complexes prepared with modified chitosan nanoparticles (gnMChi) is that the polymer:pDNA ratio is about 1:1.

The polymer:pDNA ratio of 1:2 is sufficent for the formulations prepared with nanoparticles obtained by further modification of reduced modified chitosan in order to form complexes with all pDNA.

### The Cytotoxicity of Chitosan Based Nanoparticle Formulations

The cytotoxicity tests of MChi and ARMChi were conducted with two types of cells (HEK293 and POF) by RTCA.

The chitosan is used as positive control group and PEI is used as negative control group in these tests. For the investigation of the cytotoxic concentration of the polymers, both a polymer solution with the concentration level used in transfection studies (7,5 µg/mL) and with the concentration level which are highly above of that level are analysed in order to determine the most toxic level (25 µg/mL, 50 µg/mL, 150 µg/mL). The percentage of cell viability as calculated based on time-dependent cell index values are given by RTCA.

Based on the results of the cytotoxicity studies of PEI, the negative control group, performed with HEK293 cells, followed by the addition of samples on cells at 24^{th} hour, especially at a concentration level of 7,5 µg/mL, even at 28^{th} hour, it is observed that the viability decreases to a range of 40-50%. In general, in all concentration levels used at usual working conditions a decrease of 50% is observed. At the end of the 72^{nd} hours, although the cell viability is 90% and 60% at a PEI concentration of 7,5 µg/mL and 25 µg/mL respectively, and higher toxicity is observed compared to the other modified synthesized polymers.

According to the results of the cytotoxicity studies of MChi and ARMChi on HEK293 cells, the chitosan used as positive control does not display any toxic effect on cells since it is a natural polysaccharide. In MChi polymer, no significant reduction in cell viability is observed compared to chitosan at both 28th and 72nd hours at all concentrations except at 150 µg/mL concentration. Similarly, in ARMChi polymers, the toxic effect is not seen at 7,5 µg/mL, 25 µg/mL and 50 µg/mL concentrations. However, at higher concentrations than those used in transfection studies such as 150 µg/mL, the toxic effect is observed.

According to this, in formulations prepared with both Mchi and ARMChi polymers, no toxicity is observed at concentrations below 150 µg/mL, and particularly in the concentrations of 7,5 µg/mL, 25 µg/mL and 50 µg/mL. Accordingly, in formulations prepared with the polymers of the invention, polymer concentration can be between 7 - 150 µg/mL.

The toxic effect of PEI is more clearly observed in POF cells. After the addition of PEI at 24th hour, % cell viability rates are found to be very low at 28th and 72nd hour results. On the contrary, based on the results of the cytotoxicity test, MChi and ARMChi polymers do not display any toxic effect in POF cells at all concentrations.

### The Transfection Efficiency Results of gnMChi₅ and gnARMChi₅ Nanoparticular Systems

Unmodified chitosan nanoparticles are prepared as control group. Accordingly, transfection in HEK293 cells is the highest wherein the cell number is 20000, the polymer:pDNA ratio is 2:1 and 3:1, the amount of plasmid used is 2,12 µg and the transfection efficiency is 15%. In POF cells, another type of cells used in the study, it is observed that the cell number is 20000 and no transfection is occurred.

According to the results of the transfection efficency of gnMChi₅ nanoparticles, in both types of cells used in the study, HEK293 and POF cells, the transfection is the highest wherein the cell number is 20000, the polymer:pDNA ratio is 7:1, the amount of plasmid used is 2,12 µg and the transfection efficiency is 95% for HEK293 and 20% for POF cells, respectively. Compared to Chi nanoparticles, as seen in gel electrophoresis experiment, the increase of the amine group in the molecular structure of chitosan in gnMChi polymer allows the complex formation of same amount of plasmid with smaller amount of polymeric nanoparticles. As such, higher amount of plasmid can be released into the cell with same amount of polymer. Because the amount of pDNA targeted to the nucleus increases, the amount of pDNA introduced (integrated) to genome sequencing increases as well and it is observed that GFP protein produced and both the number of transfected cells and the transfection efficiency are increased. In a review written by Kim et al. (2007) (KIM, T.H., JIANG, H.L., JERE, D., PARK, I.K., CHO, M.H., NAH, J.W., CHOI, Y.J., AKAIKE T., CHO, C.S., 2007, Chemical Modification of Chitosan as a Gene Carrier In Vitro and In Vivo, Prog. Polym. Sci., 32, 726-753), it is reported that the chitosan compounds display a quite high transfection efficiency, nonetheless the transfection efficiency is low in primary cells. It is proved that the transfection efficiency of gnMChi nanoparticles synthesized according to the present invention is high in both types of cells (HEK293 and POF).

According to the transfection efficiency results of the gnARMChi₅ nanoparticles, in both types of cells used in the study, i.e. HEK293 and POF cells, the transfection is the highest wherein the cell number is 20000, the polymer:pDNA ratio is 7:1 and 5:1 and the amount of plasmid used is 2,12 µg, the transfection efficiency is 95% for HEK293 and 20% for POF cells, respectively. Compared to the control group Chi, it is observed that depending on the modification reactions in gnARMChi polymer, the presence of reducible groups resulted with the increase in the amine groups in the molecular structure of chitosan, as seen in gel electrophoresis experiment, i.e. the same amount of pDNA can form complexes with less amount of polymeric nanoparticular system compared to chitosan and gnMChi product. Moreover, when the transfection results of gnARMChi nanoparticles are compared to that of unmodified chitosan nanoparticles, the modification applied on chitosan molecule increases the transfection in an efficient way in both cell types. Again both gnMChi nanoparticles and gnARMChi nanoparticles provide high transfection efficiency compared to MChi formulation in HEK293 cells, gnARMChi nanoparticles provide lower transfection in POF cells. Moreover, compared to gnMChi nanoparticles for the transfection efficiency, while providing a high transfection efficiency in HEK293 cells, the gnARMChi nanoparticles provide lower transfection efficiency than gnMChi nanoparticles. The main reason for this is that the ionic interaction between ARMChi polymer and pDNA is stronger than the ionic interaction between MChi polymer and pDNA.

## Claims

1. An amine modified chitosan (MChi) having the following formula: 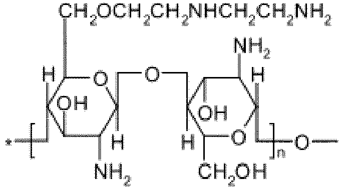 wherein n is an integer different from 0.

2. A reducible modified derivative of chitosan (RMChi) having either of the following formulas: 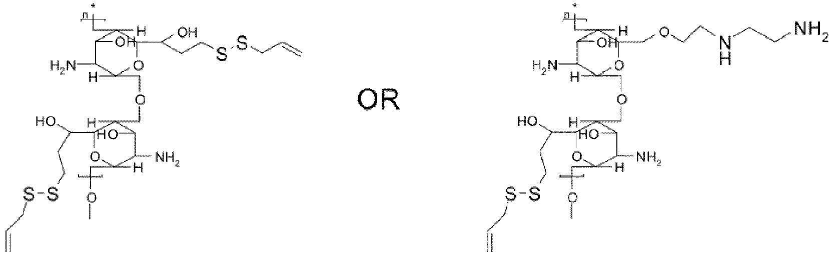 wherein n is an integer different from 0.

3. An amine modified reducible derivative of chitosan (ARMCHi) having either of the following formulas: 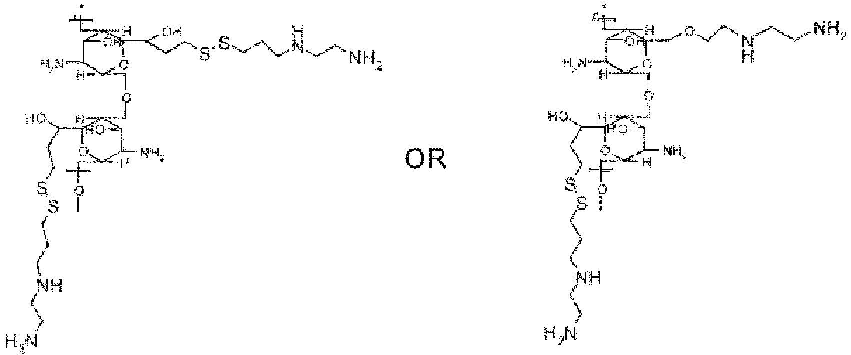 wherein n is an integer different from 0.

4. A nanoparticular form of amine modified chitosan (MChi) according to claim 1 or amine modified reducible derivative of chitosan (ARMCHi) according to claim 3 for use as a carrier in a gene therapy.

5. A nanoparticular form according to claim 4, wherein MChi or ARMChi has a particle size below 210 nm.

6. A nanoparticular form according to claim 5, wherein MChi or ARMChi has a particle size ranging from 100 to 210 nm.

7. A nanoparticular form according to claim 4, wherein polydispersity value of nanoparticular MChi or ARMChi ranges from 0.100 to 0.800.

8. A nanoparticular form according to claim 4, wherein zeta potential value of nanoparticular MChi or ARMChi ranges from 1.50 to 50.00 mV.

9. A method for producing amine modified chitosan (MChi) according to claim 1 comprising reaction of chitosan having the following formula: 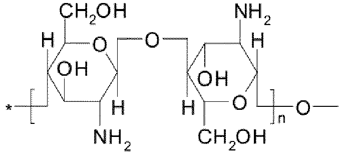
wherein n is an integer different from 0,
with N-(2-hydroxyethyl) ethylenediamine.

10. A method for producing reducible modified derivatives of chitosan (RMChi) according to claim 2, comprising reaction of a chitosan or amine modified form thereof having the following formulas: 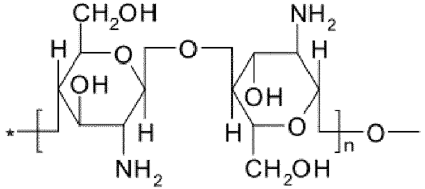 OR 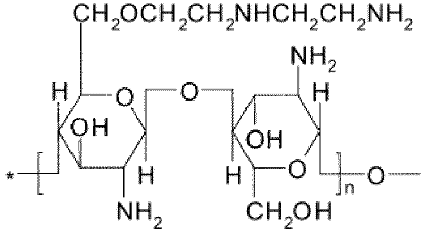
wherein n is an integer different from 0,
with an allyl disulfide compound of the formula:

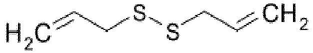

11. A method according to claim 10 comprising a gelatinization step prior to the reaction with allyl disulfide compound.

12. A method for producing amine modified reducible derivatives of chitosan (ARMCHi) according to claim 3 comprising reaction of either of the compounds according to claim 2 with ethylene diamine.

13. A method for producing nanoparticular form of an amine modified chitosan (MChi) according to claim 1 or an amine modified reducible derivative of chitosan (ARMCHi) according to claim 3 comprising preparation of nanoparticles with a particle size below 210 nm via ionic gelation method.

14. A nanoparticular form according to claim 4 which is complexed with a plasmid DNA.

15. A nanoparticular form according to claim 14 for use in gene therapy of fibroblast cells in a human or animal.

## Patentansprüche

1. Aminmodifiziertes Chitosan (MChi) mit der folgenden Formel: 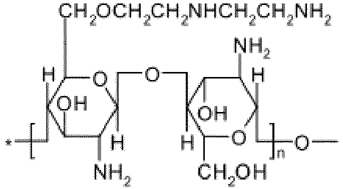 wobei n eine ganze Zahl außer 0 ist.

2. Reduzierbares modifiziertes Derivat von Chitosan (RMChi) mit einer der beiden folgenden Formeln: 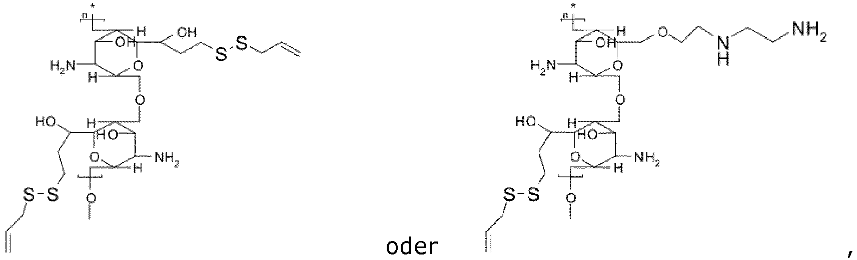 wobei n eine ganze Zahl außer 0 ist.

3. Aminmodifiziertes reduzierbares Derivat von Chitosan (ARMChi) mit einer der beiden folgenden Formeln: 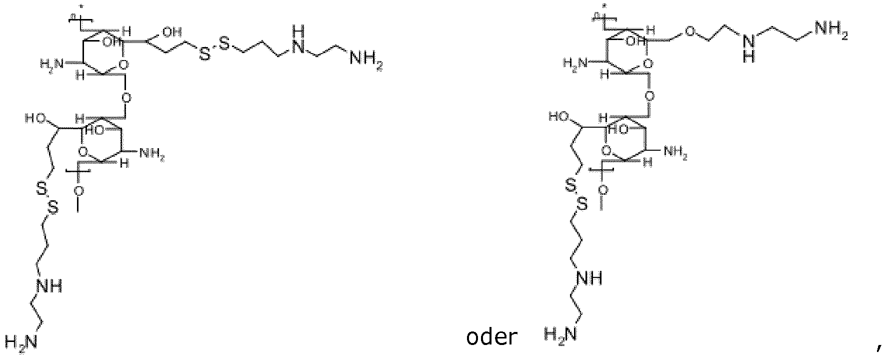 wobei n eine ganze Zahl außer 0 ist.

4. Nanopartikuläre Form von aminmodifiziertem Chitosan (MChi) gemäß Anspruch 1 oder von einem aminmodifizierten reduzierbaren Derivat von Chitosan (ARMChi) gemäß Anspruch 3 zur Verwendung als Träger in einer Gentherapie.

5. Nanopartikuläre Form gemäß Anspruch 4, wobei MChi oder ARMChi eine Teilchengröße unter 210 nm aufweist.

6. Nanopartikuläre Form gemäß Anspruch 5, wobei MChi oder ARMChi eine Teilchengröße im Bereich von 100 bis 210 nm aufweist.

7. Nanopartikuläre Form gemäß Anspruch 4, wobei die Polydispersität des nanopartikulären MChi oder ARMChi im Bereich von 0,100 bis 0,800 liegt.

8. Nanopartikuläre Form gemäß Anspruch 4, wobei das Zetapotential des nanopartikulären MChi oder ARMChi im Bereich von 1,50 bis 50,00 mV liegt.

9. Verfahren zur Herstellung von aminmodifiziertem Chitosan (MChi) gemäß Anspruch 1, umfassend die Reaktion von Chitosan mit der folgenden Formel: 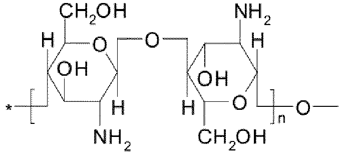
wobei n eine ganze Zahl außer 0 ist,
mit N-(2-Hydroxyethyl)ethylendiamin.

10. Verfahren zur Herstellung von reduzierbaren modifizierten Derivaten von Chitosan (RMChi) gemäß Anspruch 2, umfassend die Reaktion eines Chitosans oder einer aminmodifizierten Form davon, die die folgenden Formeln aufweisen: 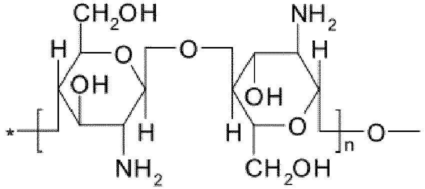 oder 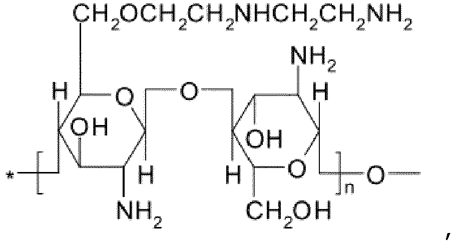
wobei n eine ganze Zahl außer 0 ist,
mit einer Allyldisulfidverbindung der Formel:

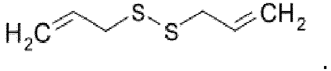

11. Verfahren gemäß Anspruch 10, umfassend einen Gelatinisierungsschritt vor der Reaktion mit der Allyldisulfidverbindung.

12. Verfahren zur Herstellung von aminmodifizierten reduzierbaren Derivaten von Chitosan (ARMChi) gemäß Anspruch 3, umfassend die Reaktion einer der Verbindungen gemäß Anspruch 2 mit Ethylendiamin.

13. Verfahren zur Herstellung einer nanopartikulären Form eines aminmodifizierten Chitosans (MChi) gemäß Anspruch 1 oder eines aminmodifizierten reduzierbaren Derivats von Chitosan (ARMChi) gemäß Anspruch 3, umfassend die Herstellung von Nanopartikeln mit einer Teilchengröße unter 210 nm durch ein ionisches Gelierungsverfahren.

14. Nanopartikuläre Form gemäß Anspruch 4, die mit einer Plasmid-DNA komplexiert ist.

15. Nanopartikuläre Form gemäß Anspruch 14 zur Verwendung in der Gentherapie von Fibroblastenzellen bei einem Menschen oder Tier.

## Revendications

1. Chitosan modifié par une amine (MChi) répondant à la formule suivante : 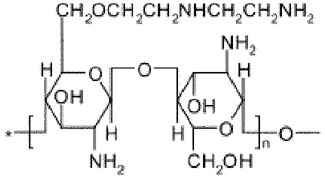 dans laquelle n est un nombre entier différent de 0.

2. Dérivé de chitosan modifié réductible (RMChi) répondant à l'une ou l'autre des formules suivantes : 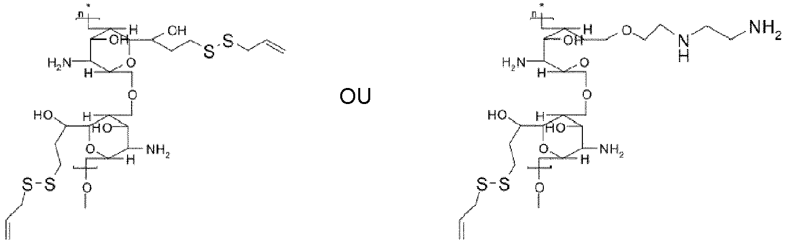 dans laquelle n est un nombre entier différent de 0.

3. Dérivé de chitosan réductible modifié par une amine (ARMCHi) répondant à l'une ou l'autre des formules suivantes : 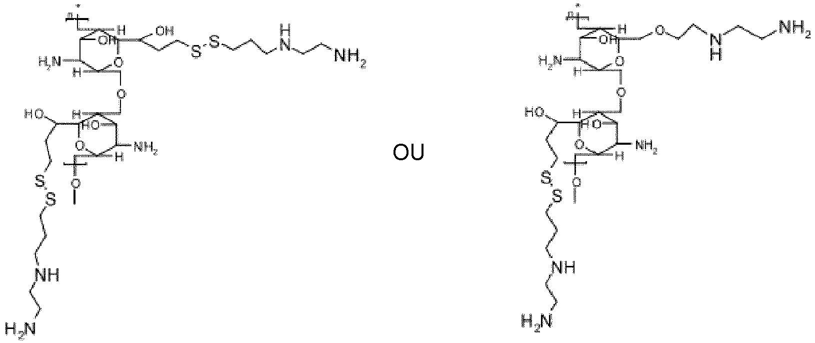 dans laquelle n est un nombre entier différent de 0.

4. Forme nanoparticulaire d'un chitosan modifié par une amine (MChi) selon la revendication 1 ou d'un dérivé de chitosan réductible modifié par une amine (ARMCHi) selon la revendication 3 pour utilisation en tant que support en thérapie génique.

5. Forme nanoparticulaire selon la revendication 4, dans laquelle le MChi ou le ARMChi a une taille de particule inférieure à 210 nm.

6. Forme nanoparticulaire selon la revendication 5, dans laquelle le MChi ou le ARMChi a une taille de particule comprise entre 100 et 210 nm.

7. Forme nanoparticulaire selon la revendication 4, dans laquelle la valeur de polydispersité du MChi ou ARMChi nanoparticulaire est comprise entre 0,100 et 0,800.

8. Forme nanoparticulaire selon la revendication 4, dans laquelle la valeur de potentiel zêta du MChi ou ARMChi nanoparticulaire est comprise entre 1,50 et 50,00 mV.

9. Procédé de production d'un chitosan modifié par une amine (MChi) selon la revendication 1 comprenant la réaction d'un chitosan répondant à la formule suivante : 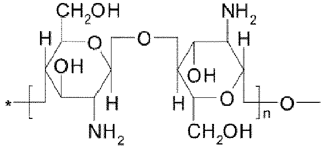
dans laquelle n est un nombre entier différent de zéro,
avec la N-(2-hydroxyéthyl)éthylène diamine.

10. Procédé de production de dérivés de chitosan modifiés réductibles (RMChi) selon la revendication 2, comprenant la réaction d'un chitosan ou d'une forme modifiée par une amine de celui-ci répondant aux formules suivantes : 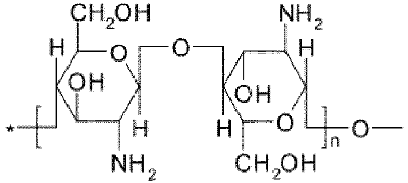 ou 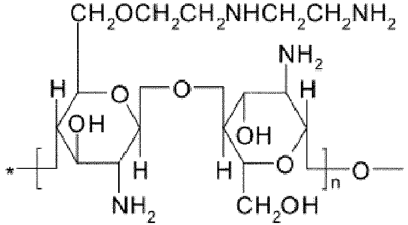
dans lesquelles n est un nombre entier différent de 0,
avec un composé de disulfure d'allyle de formule :

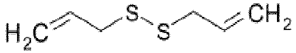

11. Procédé selon la revendication 10 comprenant une étape de gélatinisation avant la réaction avec le composé de disulfure d'allyle.

12. Procédé de production de dérivés de chitosan réductibles modifiés par une amine (ARMCHi) selon la revendication 3 comprenant la réaction de l'un ou l'autre des composés selon la revendication 2 avec l'éthylène diamine.

13. Procédé de production d'une forme nanoparticulaire d'un chitosan modifié par une amine (MChi) selon la revendication 1 ou d'un dérivé de chitosan réductible modifié par une amine (ARMCHi) selon la revendication 3 comprenant la préparation de nanoparticules ayant une taille de particule inférieure à 210 nm par un procédé de gélification ionique.

14. Forme nanoparticulaire selon la revendication 4 qui est complexée avec un ADN plasmidique.

15. Forme nanoparticulaire selon la revendication 14 pour utilisation en thérapie génique des cellules fibroblastiques chez l'homme ou l'animal.
